(19) **Europäisches Patentamt**
**European Patent Office**
**Office européen des brevets**

(11) **EP 1 877 336 B1**

(12) **EUROPÄISCHE PATENTSCHRIFT**

(45) Veröffentlichungstag und Bekanntmachung des Hinweises auf die Patenterteilung:
**16.01.2013 Patentblatt 2013/03**

(21) Anmeldenummer: **06753469.3**

(22) Anmeldetag: **02.05.2006**

(51) Int Cl.:
*C01B 3/32* *(2006.01)*       *C07C 29/154* *(2006.01)*
*C01B 3/16* *(2006.01)*       *B01J 23/80* *(2006.01)*
*B01J 23/885* *(2006.01)*

(86) Internationale Anmeldenummer:
**PCT/EP2006/004091**

(87) Internationale Veröffentlichungsnummer:
**WO 2006/117190 (09.11.2006 Gazette 2006/45)**

(54) **HERSTELLUNG VON CU/ZN/AL-KATALYSATOREN ÜBER DEN FORMIATWEG**

PRODUCTION OF CU/ZN/AL CATALYSTS VIA THE FORMATE ROUTE

PRODUCTION DE CATALYSEURS CU/ZN/AL PAR VOIE DE FORMATES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **03.05.2005 DE 102005020630**

(43) Veröffentlichungstag der Anmeldung:
**16.01.2008 Patentblatt 2008/03**

(73) Patentinhaber: **Süd-Chemie IP GmbH & Co. KG 80333 München (DE)**

(72) Erfinder:
• **POLIER, Siegfried
83052 Bruckmühl (DE)**
• **HIEKE, Martin
83052 Bruckmühl (DE)**
• **HINZE, Dieter
83059 Kilbermoor (DE)**

(74) Vertreter: **Dannenberger, Oliver Andre
Abitz & Partner
Patentanwälte
Postfach 86 01 09
81628 München (DE)**

(56) Entgegenhaltungen:
**EP-A- 0 152 809     EP-A- 0 482 753**

• **PATENT ABSTRACTS OF JAPAN Bd. 2002, Nr. 03, 3. April 2002 (2002-04-03) & JP 2001 321679 A (OSAKA GAS CO LTD), 20. November 2001 (2001-11-20) in der Anmeldung erwähnt**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Herstellung von Cu/Zn/Al-Katalysatoren.

Cu/Zn/Al-Katalysatoren, welche die Umsetzung von CO, $CO_2$ und $H_2$ zu Methanol katalysieren, sind seit längerer Zeit bekannt. Die Atomverhältnisse zwischen Kupfer und Zink können bei diesen bekannten Katalysatoren variieren, wobei jedoch das Kupfer im Allgemeinen im Überschuss vorliegt. Ferner kann ein Teil der Zinkkomponente durch Calcium, Magnesium und/oder Mangan ersetzt sein. Das als thermostabilisierende Substanz verwendete Aluminiumoxid kann teilweise auch durch Chromoxid ersetzt sein.

So ist beispielsweise aus der DE 1 965 007 ein Katalysator für die Niedertemperatur-Methanolsynthese bekannt. Für die Herstellung des Katalysators werden zunächst aus einer Lösung geeigneter Zink- und Kupfersalze durch Zugabe von Alkalimetallcarbonaten die entsprechenden basischen Carbonate ausgefällt. Diese werden von der wässrigen Phase abgetrennt, getrocknet und calciniert, um die entsprechenden Oxide zu erhalten. Die Zink- und Kupferoxide werden anschließend mit Aluminiumoxid vermischt, wobei eine Suspension der Oxide hergestellt wird, welche nicht mehr als 20 % Feststoff enthält. Diese wird anschließend homogenisiert, wobei die Homogenisierung solange durchgeführt wird, dass sich die dispergierten Oxide während 2 Stunden nicht absetzen. Nach dem Homogenisieren wird die Mischung getrocknet, tablettiert und calciniert. Um die oxidische Form in den Katalysator zu überführen, wird diese noch im Wasserstoffstrom reduziert.

Aus der DE 2 302 658 A ist ein Verfahren zur Herstellung eines Vorläufers für einen Katalysator bekannt, welcher für die Methanolsynthese verwendet werden kann. Zur Herstellung des Katalysatorvorläufers wird zunächst ein erster Niederschlag hergestellt, welcher ein zweiwertiges Metall, beispielsweise Zink, sowie ein dreiwertiges Metall, beispielsweise Aluminium, in Form einer Verbindung enthält, die sich thermisch zu den entsprechenden Oxiden zersetzen lässt. Geeignete Verbindungen sind z.B. Carbonate oder Bicarbonate. Ferner wird ein zweiter Niederschlag hergestellt, welcher Kupferverbindungen enthält, die sich thermisch zu Oxiden zersetzen lassen. Die beiden Niederschläge werden vermischt. Es schließen sich die üblichen Stufen der Trocknung und Calcinierung an, um aus den Metallverbindungen die Oxide zu erzeugen und möglicherweise die Ausbildung einer Spinellstruktur zu bewirken. Der Feststoff wird dann tablettiert. Um den Vorläufer in einen aktiven Katalysator zu überführen, werden die Tabletten im Wasserstoffstrom reduziert.

In der DE 2 056 612 A wird ein Verfahren zur Herstellung von Methanol beschrieben, wobei die Umsetzung an einem Katalysator erfolgt, welcher Zink, Kupfer und Aluminium enthält. Der Katalysator gehört einer Mischkristallreihe der Formel $(Cu_xZn_y)Al_2(OH)_{16}$ x $CO_3$ x $4H_2O$ an, in welcher x und y Zahlenwerte von 0,5 bis 5,5 annehmen können, und die Summe von x und y gleich 6 ist. Die Mischkristallverbindung wird aus einer wässrigen Lösung, die Kupfer-, Zink- und Aluminiumsalze enthält, durch Zugabe von Alkalicarbonaten, Alkalibicarbonaten oder deren Mischungen ausgefällt. Das Atomverhältnis der Summe der zweiwertigen Metalle Kupfer und Zink zum dreiwertigen Aluminium in der Mischkristallreihe ist konstant und beträgt 6:2. Für die Herstellung werden Kupfer, Zink und Aluminium in Form geeigneter Salze, vorzugsweise der Nitrate, in Wasser gelöst, und zwar in einem Mengenverhältnis, das der gewünschten Zusammensetzung des Katalysators entspricht. Diese Lösung wird auf Temperaturen von 50 bis 100 °C, vorzugsweise 70 bis 100 °C, erhitzt, und mit einer entsprechend temperierten wässrigen Lösung eines Fällungsmittels, beispielsweise eines Alkalicarbonats, behandelt. Der entstandene Niederschlag wird abfiltriert, gewaschen und getrocknet. Die getrocknete Verbindung wird bei Temperaturen im Bereich von 200 bis 500 °C während 12 bis 24 h calciniert. Das calcinierte Produkt wird zu Tabletten geformt und anschließend durch Reduktion im Wasserstoffstrom in die aktive Form des Katalysators überführt.

In der US 4,279,781 wird ein Katalysator für die Methanolsynthese beschrieben, welcher Kupfer- und Zinkoxid sowie ein Metalloxid für die thermische Stabilisierung, beispielsweise Aluminiumoxid, umfasst. Das Verhältnis von Kupferoxid und Zinkoxid beträgt, berechnet als Gewicht der Metalle, 2:1 bis 3,5:1. Die Herstellung des Katalysators erfolgt durch gemeinsame Fällung löslicher Zink-, Kupfer- und Aluminiumsalze, beispielsweise der Nitrate. Dadurch wird eine innige Durchmischung der Katalysatorbestandteile erreicht. Um die aktive Form zu erhalten, wird der Katalysatorvorläufer im Wasserstoffstrom reduziert.

Aus der EP 0 125 689 A2 ist ein Katalysator für die Methanolsynthese bekannt, welcher als katalytisch wirksame Substanzen Kupferoxid und Zinkoxid und als thermostabilisierende Substanz Aluminiumoxid enthält. Der Katalysator zeichnet sich durch eine bestimmte Porenradienverteilung aus, wobei der Anteil der Poren mit einem Durchmesser von 20 bis 75 Å (Mesoporen) mindestens 20 % und der Anteil der Poren mit einem Durchmesser von mehr als 75 Å (Makroporen) höchstens 80 % beträgt. Die gewünschte Porenradienverteilung kann dadurch erreicht werden, dass bei der Herstellung des Katalysators kolloidal verteiltes Aluminiumoxid oder -hydroxid verwendet wird. Für die Herstellung dieser Katalysatoren wird die katalytisch wirksame Kupferoxid-Zinkoxid-Komponente aus wässrigen Lösungen der entsprechenden Salze, z.B. den Nitraten, Sulfaten, Chloriden, Acetaten, mit alkalisch reagierenden Substanzen in Gegenwart eines kolloidal verteilten Aluminiumoxids oder -hydroxids ausgefällt. Das Fällungsprodukt kann anschließend getrocknet, calciniert, zu Formkörpern verpresst und gegebenenfalls reduziert werden.

Aus der EP 0 152 809 A2 ist ein Katalysator zur Synthese von Methanol und höhere Alkohole enthaltenden Alkoholgemischen bekannt, welcher in Form eines oxidischen Vorläufers Kupferoxid und Zinkoxid enthält, die durch eine Reduktion wenigstens eines Teils des Kupferoxids in eine katalytisch wirksame Komponente überführbar sind, sowie Aluminiumoxid als thermostabilisierende Substanz, und mindestens ein Alkalicarbonat bzw. Alkalioxid. Der oxidische Vorläufer weist einen Anteil an Poren mit einem Durchmesser zwischen 14 und 7,5 nm von 20 bis 70 % des Gesamtvolumens auf. Der Alkaligehalt beträgt 13 bis 130 x $10^{-6}$ Grammatom Alkalimetall je Gramm des oxidischen Vorläufers. Die Aluminiumoxidkomponente wurde aus einem kolloidal verteilten Aluminiumhydroxid erhalten. Für die Herstellung des Katalysators verwendet man im Allgemeinen Lösungen der Nitrate von Kupfer und Zink und führt die Fällung vorzugsweise mit einer wässrigen $K_2CO_3$-Lösung durch. Die Konzentration der Lösung beträgt vorzugsweise 5 bis 20 Gew.- %. Statt von den Nitraten kann auch von den entsprechenden Metallformiaten oder -acetaten ausgegangen werden. Die Fällung kann auch mit Hilfe einer Kaliumhydrogencarbonatlösung durchgeführt werden. Die Fällung kann ansatzweise oder kontinuierlich durchgeführt werden. Vorzugsweise wird die Fällung durch kontinuierliche Zusammenführung der das kolloidal verteilte Aluminiumhydroxid enthaltenden Lösung der Nitrate von Kupfer und Zink mit wässriger $K_2CO_3$-Lösung durchgeführt. Anschließend an die Fällung wird der gewaschene Niederschlag des Katalysators calciniert und durch Behandlung mit einer Lösung einer Alkalimetallverbindung alkalisiert. Die alkalisierten Katalysatorvorläufer werden nach dem Trocknen in an sich bekannter Weise zu Formkörpern verpresst, wobei Gleitmittel, wie Graphit, zugesetzt werden können. Um den Katalysatorvorläufer in eine aktive Form überzuführen, wird dieser mit Wasserstoff reduziert.

Aus der WO 03/053569 A1 ist ein Katalysator für die Methanolsynthese bekannt, welcher Kupferoxid und Zinkoxid als katalytisch wirksame Substanzen sowie Aluminiumoxid als thermostabilisierende Substanz enthält. Zur Herstellung des Katalysators werden aus einer Lösung, welche die Cu- und Zn-Salze sowie einen Teil der Al-Salze enthält, mit einer Alkalicarbonat- oder Alkalialuminatlösung die entsprechenden Hydroxocarbonate bzw. Hydroxide ausgefällt. Entweder die Lösung der Cu- und Zn-Salze oder die Alkalicarbonat- oder Alkalialuminatlösung enthält ein Aluminiumhydroxidsol. Der erhaltene Niederschlag wird von der Fällungslösung abgetrennt, gewaschen, getrocknet und gegebenenfalls calciniert. Bei der Herstellung des Katalysators geht man vorzugsweise von den Kupfer- und Zinknitraten aus, welche vorzugsweise mit Natriumcarbonat bzw. Natriumaluminat ausgefällt werden.

In der JP 2001-144779 wird ein Kupfer-Zink-Katalysator für die Reaktion von Kohlenmonoxid und Wasser zu Kohlendioxid und Wasser beschrieben, welcher hergestellt wird, indem eine Lösung, welche Kupferformiat und Zinkformiat enthält, mit einer wässrigen Lösung eines Alkalimaterials gemischt wird. Der dabei erhaltene Niederschlag wird anschließend filtriert, gewaschen, getrocknet und calciniert. Der calcinierte Feststoff wird durch Beigabe von Wasser aufgeschlämmt und auf die Oberfläche eines wabenförmigen Trägers aufgetragen. Als Bindemittel kann Aluminiumoxidsol oder Zirkonoxidsol verwendet werden. Diese werden der Aufschlämmung des calcinierten Niederschlags beigegeben. Das Aluminiumoxid ist daher nicht homogen im Katalysator verteilt sondern nur zwischen den Katalysatorpartikeln angeordnet. Es hat also lediglich die Aufgabe als Bindemittel zwischen den kupfer- und zinkhaltigen Katalysatorpartikeln sowie dem Wabenträger zu wirken und ist nicht aktiver Bestandteil des Katalysators.

Für die Herstellung von Cu/Zn/Al-Katalysatoren für die Methanolsynthese werden bei technisch realisierten Verfahren wegen ihrer guten Wasserlöslichkeit meist Kupfer- und Zinknitrate verwendet. Bei der Ausfällung fallen daher Abwässer an, die große Mengen an Natriumnitrat enthalten. Bei der Einleitung in Oberflächengewässer würde dies zu einer Überdüngung führen. Vor der Einleitung in Oberflächengewässer muss daher der Gehalt an wasserlöslichem Stickstoff im bei der Herstellung des Methanolsynthesekatalysators anfallenden Abwasser drastisch reduziert werden.

Aufgabe der Erfindung ist es daher, ein Verfahren zur Herstellung von Cu/Zn/Al-Katalysatoren zur Verfügung zu stellen, welches einerseits eine deutliche Verringerung der Salzfracht, insbesondere an Alkalimetallnitraten, im Abwasser ermöglicht und welches andererseits Katalysatoren für die Methanolsynthese zur Verfügung stellt, welche mindestens eine vergleichbare Aktivität wie ein aus Metallnitraten hergestellter Katalysator aufweisen.

Diese Aufgabe wird mit einem Verfahren mit den Merkmalen des Patentanspruchs 1 gelöst. Vorteilhafte Ausführungsformen des Verfahrens sind Gegenstand der abhängigen Ansprüche.

Beim erfindungsgemäßen Verfahren zur Herstellung von Cu/Zn/Al-Katalysatoren wird zunächst eine erste wässrige Lösung hergestellt, welche zumindest Kupferformiat und Zinkformiat enthält. Weiter wird eine zweite Lösung hergestellt, welche ein Fällungsmittel enthält. Unter einem Fällungsmittel wird dabei ein Reagenz verstanden, welches, direkt oder indirekt, Ionen bereitstellt, beispielsweise Hydroxylionen und/oder Carbonationen, mit welchen die Metalle, insbesondere Kupfer, Zink und Aluminium, ausgefällt werden können. Die erste Lösung und gegebenenfalls die zweite Lösung enthält ein Aluminiumhydroxidsol/gel-Gemisch. Unter einem Aluminiumhydroxidsol wird dabei eine feindisperse Verteilung von Aluminiumhydroxid in Wasser verstanden, in welcher sich bereits Polysäuren durch Kondensation von Aluminiumhydroxid ausgebildet haben, in der wässrigen Phase mit bloßem Auge jedoch noch keine Partikel erkennbar sind, also eine klare Lösung vorliegt. Unter einem Aluminiumhydroxidgel wird eine Dispersion von Aluminiumhydroxid in Wasser verstanden, wobei sich bereits größere Agglomerate aus Polysäuren gebildet haben, sodass auch mit bloßem Auge Partikel wahrgenommen werden können, beispielsweise als Trübung der wässrigen Phase.

In einem Fällungsschritt werden die erste Lösung und die zweite Lösung vereinigt, wobei ein Niederschlag erhalten wird. Der Niederschlag wird von der wässrigen Phase abgetrennt, wobei die wässrige Phase ein Abwasser bildet, welches einer Aufarbeitung zugeführt wird.

Der Niederschlag wird gewaschen, bis er einen Alkaligehalt von weniger als 500 ppm bezogen auf den bei 600°C geglühten Katalysator aufweist. Anschließend wird der Niederschlag getrocknet, ggf. calciniert und gemahlen.

Beim erfindungsgemäßen Verfahren fallen keine nitrathaltigen Abwässer an. Durch die Verwendung von Kupferformiat und Zinkformiat als wasserlösliche Kupfer- und Zinksalze enthält das Abwasser Formiationen, welche in einfacher Weise aufgearbeitet werden können. Durch die Verwendung von Formiaten wird die Belastung des Abwassers mit organischem Material relativ niedrig gehalten. Dies ist ein Vorteil gegenüber der Verwendung höherer Carbonsäuren, wie Essigsäure, da diese durch die größere Anzahl von C-H-Bindungen die organische Belastung im Abwasser erhöhen. Als weiterer Vorteil ist Ameisensäure, welche für die Herstellung der Kupfer- und Zinkformiate benötigt wird, kostengünstig herzustellen, so dass das erfindungsgemäße Verfahren auch unter Kostengesichtspunkten vorteilhaft ist.

Für das erfindungsgemäße Verfahren ist wesentlich, dass zumindest ein Teil des Aluminiums in Form eines Aluminiumhydroxidsols und ein anderer als Aluminiumhydroxidgel in die Fällungslösung eingebracht wird. Bei Verzicht auf die Zugabe des Aluminiumhydroxidsol/-gel-Gemisches in die Lösung der Metallsalze sinkt die Gewicht-Zeit-Ausbeute (GZA, [kg Methanol/{kg Katalysator x Stunde}]).

Der Niederschlag wird nach der Abtrennung sorgfältig gewaschen, so dass sein Alkaligehalt, bezogen auf den bei 600 °C geglühten oxidischen Katalysator auf Werte von weniger als 500 ppm, vorzugsweise weniger als 400 ppm, insbesondere auf Werte im Bereich von 100 bis 300 ppm, absinkt. Das dabei anfallende Waschwasser kann mit dem formiathaltigen Abwasser vereinigt und gegebenenfalls aufgearbeitet werden. Nach dem Waschen, Trocknen und ggf. Calcinieren weist die oxidische Form des Katalysators noch einen restlichen Formiatgehalt von weniger als 5 Gew.- %, vorzugsweise 0,5 bis 4 Gew.- %, insbesondere bevorzugt 1 - 2 Gew.- % auf. Der Formiatgehalt lässt sich beispielsweise durch oxidative Titration oder durch quantitative chromatographische Verfahren, beispielsweise HPLC, bestimmen.

Neben dem Kupferformiat und dem Zinkformiat kann die erste wässrige Lösung auch noch übliche Promotoren enthalten, wie beispielsweise Calcium, Magnesium, Mangan, Cer, Lanthan, sowie Ruthen oder Palladium. Neben den genannten Promotoren können auch noch andere Promotoren verwendet werden. Die Promotoren werden vorzugsweise ebenfalls in Form ihrer Formiate vorzugsweise in die erste wässrige Lösung eingebracht. Ihr Anteil an der oxidischen Form des Katalysators beträgt, berechnet als Oxid, vorzugsweise weniger als 10 Gew.- %, insbesondere weniger als 5 Gew.- %. Werden Edelmetalle, wie Ruthenium oder Palladium als Promotoren eingesetzt, sind diese vorzugsweise in Mengen von weniger als 1 Gew.- % enthalten.

Wie bereits erwähnt, enthält beim erfindungsgemäßen Verfahren die erste und gegebenenfallt die zweite Lösung ein Aluminiumhydroxidsol/- gel-Gemisch. Die aus dem Aluminiumhydroxidsol/-gel entstehenden Folgeprodukte dienen sowohl als Träger als auch als thermostabilisierende Substanz. Ohne an diese Theorie gebunden zu sein vermuten die Erfinder, dass aus dem Aluminiumhydroxidsol/-gel-Gemisch beim Erhitzen ein dreidimensionales Netzwerk entsteht, in dessen Lücken die zu den aktiven Spezies gehörenden, nach der Reduktion vorhandenen Kupferkristallite angeordnet sind. Dadurch wird ein weiteres Zusammenwachsen der Kupferkristallite während der Methanolsynthese erschwert, was die Stabilität des Katalysators und seine Standzeit in technischen Prozessen erhöht.

Vom Zinkoxid wird angenommen, dass es zum Einen einen wichtigen Einfluss auf die Ausbildung der aktiven Spezies ausübt und zum Anderen durch seine teilweise nadelförmige Struktur zur Stabilität des Katalysators beiträgt. Außerdem wirkt das Zinkoxid als Giftfänger, indem es mit etwa vorkommenden Schwefelverbindungen reagiert.

Die ggf. als Promotoren enthaltenen Oxide des Calcium, Magnesium, Mangan, Cer und des Lanthan wirken ebenfalls stabilisierend.

Die erste Lösung, die ein Gemisch verschiedener Metallsalze enthält, wird hergestellt, indem

- eine wässrige Kupferformiatlösung hergestellt wird, indem ein Kupfersalz durch Zugabe von Ameisensäure rückstandsfrei gelöst wird,
- eine wässrige Dispersion oder Lösung eines Zinksalzes hergestellt wird,
- eine wässrige Aluminiumsalzlösung hergestellt wird, und
- die Kupferformiatlösung, die Dispersion oder Lösung des Zinksalzes und die Aluminiumsalzlösung vereinigt werden.

Sofern der Cu/Zn/Al-Katalysator noch durch Promotoren modifiziert werden soll, können diese beispielsweise der ersten Lösung zugegeben werden. Die Promotoren können in Form geeigneter Salze, beispielsweise als Carbonate, Oxide oder Hydroxide, zugegeben werden. An sich können diese Salze zu einem beliebigen Zeitpunkt zugegeben werden, also zur Kupferformiatlösung, zur Dispersion oder Lösung des Zinksalzes, oder auch nach Vereinigung der Kupferformiatlösung und der Dispersion oder Lösung des Zinksalzes. Besonders teure Promotoren, wie Edelmetalle, werden bevorzugt in einem späteren Verfahrensschritt, beispielsweise vor der Sprühtrocknung, der Suspension zugesetzt, oder nach der Sprühtrocknung feinstverteilt auf das trockene Pulver gesprüht. Bei der Herstellung der Kupfer-Zinkformiatlösung wird soviel Ameisensäure zugegeben, dass bezogen auf die eingesetzte Menge an Kupfer- und Zinksalz unter Berücksichtigung der Stöchiometrie die Ameisensäure in einem Überschuss von mindestens 10 Mol- %, bevorzugt 10 bis 20 Mol- %, insbesondere bevorzugt 14 bis 16 Mol- % vorliegt. Vorzugsweise beträgt der pH-Wert der Kupferformiatlösung nach der Zugabe der Ameisensäure weniger als 3, vorzugsweise weniger als 2,5.

Die Lösung oder Dispersion des Zinksalzes wird mit der Kupferformiatlösung vereinigt. Nach Vereinigung der Kupferformiat- und der Zinksalzlösung bzw. -dispersion liegen dann sowohl das Kupfer als auch das Zink in Form ihrer Formiate in Lösung vor. Die erhaltene Lösung weist einen pH-Wert im Bereich von 3,0 bis 4,0, insbesondere bevorzugt 3,5 bis 3,7 auf. Anschließend wird die Aluminiumsalzlösung zur Kupfer/Zinklösung gegeben.

Die Aluminiumsalzlösung wird in mehreren Teilen zur Kupfer/Zink-Lösung gegeben. Dabei wird zumindest ein erster Anteil der Aluminiumsalzlösung in der Weise hergestellt, dass zumindest ein erster Teil des Aluminiumsalzes unter Zugabe von Ameisensäure in Wasser gelöst wird.

Bei der Herstellung des ersten Anteils der Aluminiumsalzlösung wird in der Weise vorgegangen, dass beispielsweise Natriumaluminat zunächst in Wasser gelöst wird und dann so viel Ameisensäure zugegeben wird, bis der pH-Wert im Bereich von weniger als 5, vorzugsweise 4,5 bis 2, insbesondere 4 bis 3 liegt. Es wird bevorzugt soviel Ameisensäure zugegeben, dass eine klare Lösung erhalten wird.

Weiter wird ein zweiter Anteil der Aluminiumsalzlösung hergestellt, indem ein zweiter Anteil des Aluminiumsalzes in Wasser gelöst wird. Dem zweiten Anteil der Aluminiumsalzlösung wird dabei keine Ameisensäure zugesetzt. Zur Fertigstellung der ersten Lösung, die das Gemisch aller Metallsalze vor der Fällung enthält, wird dann der erste Anteil der Aluminiumsalzlösung und der zweite Anteil der Aluminiumsalzlösung zeitlich versetzt, zu der wässrigen Kupfer/Zink-Lösung gegeben.

Der zweite Anteil der Aluminiumsalzlösung wird beispielsweise hergestellt, indem $NaAl_2$ in Wasser gelöst wird. Der pH-Wert der wässrigen $NaAlO_2$-Lösung liegt abhängig vom Alkaliüberschuss im Edukt im Bereich von 11 bis 14, vorzugsweise 12 bis 13.

Die Anteile des ersten und zweiten Teils der Aluminiumsalzlösung können, bezogen auf den Aluminiumgehalt im Bereich von 1 : 99 bis 99 : 1, besonders bevorzugt 30 : 70 bis 70 : 30 und insbesondere bevorzugt zu etwa 50 : 50 gewählt werden.

Die Herstellung der Aluminiumsalzlösungen erfolgt vorzugsweise bei Temperaturen von weniger als 40 °C, insbesondere bevorzugt weniger als 30 °C. Diese Temperatur sollte auch nicht überschritten werden, wenn die Aluminiumsalzlösung zur Kupfer/Zink-Lösung oder zur Kupferformiatlösung bzw. zur Dispersion oder Lösung des Zinksalzes gegeben wird. Auf diese Weise wird die Ausbildung von grobdispersen polymeren Aluminiumverbindungen unterdrückt. Unter grobdispersen polymeren Aluminiumverbindungen werden komplexe Aluminiumhydroxoverbindungen verstanden, welche mit dem Auge sichtbare Partikel ausbilden, welche relativ rasch absinken. Vorzugsweise erfolgt daher die Herstellung der Lösungen in einem Kessel, welcher mit einer entsprechenden Kühlvorrichtung ausgerüstet ist.

Aluminiumsalze, welche geeignet im erfindungsgemäßen Verfahren eingesetzt werden können, sind beispielsweise Aluminiumdi- und -triformiat, $Al(NO_3)_3$-Hydrate oder $NaAlO_2$. Die Aluminiumsalzlösungen weisen vorzugsweise eine Aluminiumkonzentration im Bereich von etwa 0,4 bis etwa 1,1 Mol/l, insbesondere bevorzugt etwa 0,9 bis 1,1 Mol/l auf. Der obere Wert des angegebenen Bereichs wird dabei durch die Löslichkeitsgrenze des Aluminiumsalzes bestimmt, während sich die untere Grenze aus wirtschaftlichen Überlegungen ergibt.

Als Kupfersalze werden bevorzugt solche Salze verwendet, deren Anionen Oxide, Hydroxide und Carbonate oder deren durch Reduktion erhaltenen Abkömmlinge sind und in der ersten Lösung bzw. in der oxidischen Form des Katalysators nicht mehr separat über unterscheidbare Elemente nachzuweisen sind. Bevorzugt wird das Kupfersalz ausgewählt aus $CuO$, $Cu(OH)_2$ und $Cu(OH)_2$ x $CuCO_3$.

Als Zinksalz wird vorzugsweise ebenfalls eine Zinkverbindung gewählt, deren Anion in der ersten Lösung bzw. in der oxidischen Vorstufe des Katalysators nicht mehr störend, bevorzugt als unterscheidbares Element nicht mehr nachweisbar ist. Vorzugsweise wird als Zinksalz ZnO gewählt.

Die Konzentration der Kupferformiatlösung wird vorzugsweise so gewählt, dass nach der Vereinigung von Kupferformiatlösung und Zinksalzlösung bzw. -dispersion die Konzentration des Kupfers in einem Bereich von etwa 0,1 bis etwa 0,5, insbesondere bevorzugt 0,3 bis etwa 0,5 Mol/l, eingestellt ist. Der obere Wert wird dabei durch die Löslichkeitsgrenze des Kupfersalzes bestimmt, während sich die untere Grenze aus wirtschaftlichen Überlegungen ergibt, da die Verarbeitung verdünnter Lösungen größere Volumina ergibt, was beispielsweise die Dimensionierung der Vorrichtungen beeinflusst, in welchen das erfindungsgemäße Verfahren durchgeführt wird.

Die Konzentration des Zinksalzes wird vorzugsweise so gewählt, dass nach der Vereinigung von Kupferformiatlösung und Zinksalzlösung bzw. -dispersion die Konzentration des Zinks vorzugsweise im Bereich von etwa 0,1 bis etwa 0,2 Mol/l, vorzugsweise 0,15 bis etwa 0,2 Mol/l liegt. Die obere Grenze ergibt sich auch hier aus der Löslichkeit des Zinksalzes und die untere Grenze aus wirtschaftlichen Überlegungen.

Die Aluminiumsalzlösung wird zur Kupfer/Zink-Lösung gegeben. Der pH der ersten Lösung, die vorzugsweise die Gesamtmenge an Cu, Zn und Al enthält, wird dann vorzugsweise auf einen Wert im Bereich von 4,0 bis 5,0, besonders bevorzugt 4,2 bis 4,4 eingestellt.

Als Fällungsreagenz wird vorzugsweise eine Alkalimetallbase verwendet. Als Alkalimetallbase werden vorzugsweise Alkalimetallcarbonate, Alkalimetallhydrogencarbonate oder Alkalimetallaluminate eingesetzt. Als Alkalimetall wird vorzugsweise Natrium verwendet. Wird beispielsweise eine Sodalösung als Fällungsreagenz eingesetzt, so hat die Sodalösung bevorzugt eine Konzentration von 80 g/l bis 200 g/l, vorzugsweise 170 bis 180 g/l.

Gemäß einer weiteren Ausführungsform des erfindungsgemäßen Verfahrens wird als Fällungsreagenz Wasserstoffperoxid verwendet. Das Wasserstoffperoxid wird zur ersten Lösung bzw. Dispersion gegeben, welche Kupfer, Zink und Aluminium in Form ihrer Formiate bzw. Hydroxoformiate enthält. Durch das Wasserstoffperoxid wird das Formiat zu Carbonat oxidiert, so dass die Metalle in Form ihrer Hydroxocarbonate, Carbonate bzw. Hydroxide ausgefällt werden. Bei der Oxidation des Formiatanions wird zunächst das Hydrogencarbonatanion und nachfolgend das Carbonatanion bei ansteigendem pH-Wert gebildet. Die eingesetzten Metallionen werden in einer sequentiellen Fällung in der Reihe Al-Cu-Zn als Hydroxocarbonate abgeschieden. Der Einsatz einer carbonathaltigen Alkalilösung kann somit entfallen. Neben Wasserstoffperoxid können auch andere geeignete Oxidationsmittel verwendet werden, beispielsweise Ozon.

Zur Herstellung der oxidischen Katalysatorvorstufe werden erste und zweite Lösung zunächst vereinigt, wobei ein Niederschlag erhalten wird. Die Fällung wird bevorzugt in der Weise durchgeführt, dass während der Fällung ein pH-Wert im Bereich von 3,5 bis 7,5 vorzugsweise 6,0 bis 7,0 insbesondere bevorzugt 6,5 ± 0,1, eingehalten wird.

Die Temperatur wird während der Fällung vorzugsweise in einem Bereich von 25 bis 95 °C, insbesondere bevorzugt 50 bis 75 °C, gehalten.

Nach dem Vermischen wird der entstandene Niederschlag vorzugsweise gealtert. Dazu kann die beim Vermischen von erster und zweiter Lösung erhaltene Suspension beispielsweise in ein Alterungsgefäß übergeführt werden, in welchem die Suspension beispielsweise mit einem geeigneten Rührwerk bewegt werden kann.

Die Alterung wird vorzugsweise während einer Dauer von 10 Minuten bis 10 Stunden, insbesondere bevorzugt 1 bis 5 Stunden, durchgeführt.

Während des Alterns wird die Suspension vorzugsweise temperiert, wobei die Alterung insbesondere bei einer Temperatur von mehr als 60 °C, insbesondere bevorzugt im Bereich von 65 bis 80 °C, erfolgt.

Die Vereinigung von erster und zweiter Lösung wird vorzugsweise in der Weise durchgeführt, dass die Lösungen parallel in ein Mischgefäß eingeleitet und dort vermischt werden. In diesem erfolgt beispielsweise mittels eines geeigneten Rührwerks eine rasche Vermischung.

Vorzugsweise wird die Fällung jedoch als kontinuierliche Fällung durchgeführt. Dazu wird ein entsprechend dimensioniertes Mischgefäß vorgesehen, in welches erste und zweite Lösung kontinuierlich zugeführt und die entstandene Mischung kontinuierlich abgeführt wird. Vorzugsweise wird das Volumen des Mischgefäßes so gewählt, dass eine kontinuierliche Zuleitung von erster und zweiter Lösung in das Mischgefäß und eine kontinuierliche Ableitung der Mischung erfolgen kann, wobei die Aufenthaltsdauer der Mischung vorzugsweise im Bereich von etwa 0,1 Sekunden bis 10 Minuten, besonders bevorzugt etwa 1 bis 120 Sekunden, insbesondere bevorzugt 1 bis 20 Sekunden beträgt.

Die Aufenthaltsdauer der Mischung im Mischungsgefäß hängt stark von der Dimensionierung des Mischungsgefäßes und der Strömungsgeschwindigkeit ab. Die Dimensionierung des Mischbehälters sowie die Zu- und Abflussraten der Lösungen bzw. Suspension kann vom Fachmann entsprechend geeignet gewählt werden.

Nach der Fällung und einem gegebenenfalls durchgeführten Alterungsschritt wird der Niederschlag von der wässrigen Phase abgetrennt, wozu übliche Verfahren verwendet werden können, beispielsweise eine Filtration. Der Niederschlag wird anschließend gewaschen und getrocknet. Vorzugsweise wird der Niederschlag nach dem Trocknen calciniert. Die Calcinierung erfolgt abhängig vom verwendeten Verfahren bei Temperaturen von vorzugsweise 140 °C bis 1000 °C, insbesondere bevorzugt 170 °C bis 350 °C für eine Dauer von wenigstens 0,1 Sekunden, bevorzugt wenigstens 4 Minuten, besonders bevorzugt 20 Minuten bis 8 Stunden, insbesondere bevorzugt 30 Minuten bis 4 Stunden. Abhängig von den gewählten Calcinierbedingungen wird das im Filterkuchen verbliebene Formiat beim Calcinieren oxidativ unter Luft oder durch eine intramolekulare Redoxreaktion unter Inertgas weitgehend eliminiert. Im letzteren Fall wird das $Cu(HCO_2)_2$ x $H_2O$ vermutlich zunächst zu $H_2$ und $CuC_2O_4$ zersetzt. Das Kupferoxalat reagiert dann weiter zu $CO_2$ und elementarem Kupfer. Die Calcinierung kann in üblichen Vorrichtungen durchgeführt werden. Bei einer industriellen Fertigung werden wegen des besseren Wärmeübergangs sowohl Pulsations- als auch Wirbelbettreaktoren wie die kommerziell bevorzugten Drehrohröfen verwendet. Pulsationstrockner ermöglichen sehr kurze Trockenzeiten im Bereich von weniger als 1 Sekunde, allgemein im Bereich von 0,1 Sekunden bis 4 Minuten, wobei sehr hohe Temperaturen von bis zu 1000 °C zur Anwendung gelangen können.

Das calcinierte Pulver kann gegebenenfalls gemahlen werden und dann mittels üblicher Werkzeuge beispielsweise zu Tabletten oder Extrudaten verarbeitet werden. Es ist jedoch auch möglich, das Pulver aufzuschlämmen, auf eine sehr feine Korngröße zu mahlen und mit der erhaltenen Suspension geeignete Trägerkörper, beispielsweise Waben, zu beschichten. Hier können übliche Verfahren angewendet werden. Die Korngröße wird dabei geeignet so eingestellt, dass die mittlere Korngröße $D_{50}$ im Bereich von 10 nm bis 10 $\mu$m, insbesondere bevorzugt 100 nm bis 5 $\mu$m liegt. Die mittlere Korngröße lässt sich beispielsweise durch Laserlichtbeugung bestimmen. Geeignete Katalysatoren lassen sich beispielsweise mit Korngrößen herstellen, bei denen der $D_{50}$-Wert im Bereich von etwa 2 - 3 $\mu$m liegt.

Ein besonderer Vorteil des erfindungsgemäßen Verfahrens liegt darin, dass das nach der Abtrennung des Niederschlags anfallende formiathaltige Abwasser mit vergleichsweise einfachen Mitteln aufgearbeitet werden kann. Dazu wird das formiathaltige Abwasser vorzugsweise einer oxidativen Behandlung unterworfen, wobei die Formiationen in wässriger Lösung pH-abhängig im Wesentlichen zu Carbonat, Hydrogencarbonat, Kohlendioxid und Wasser oxidiert werden. Der Gehalt des Abwassers an Formiat, beispielsweise in Form von Natriumformiat, liegt bei einer technischen Umsetzung des erfindungsgemäßen Verfahrens üblicherweise im Bereich von 0,2 bis 1,5 Mol/l, insbesondere bevorzugt 0,8 bis 1,0 Mol/l, wobei jedoch auch höhere oder niedrigere Formiatkonzentrationen vorliegen können. Durch die oxidative Behandlung des Abwassers kann die Formiatkonzentration auf Werte von weniger als 0,1 Mol/l, vorzugsweise 0,01 bis 0,075 Mol/l, insbesondere bevorzugt 0,02 bis 0,04 Mol/l abgesenkt werden. Dies entspricht einer Absenkung der im Abwasser enthaltenen Formiatmenge um mehr als 95 %.

Gemäß einer bevorzugten Ausführungsform wird zur oxidativen Behandlung Wasserstoffperoxid zum formiathaltigen Abwasser gegeben. Das Wasserstoffperoxid wird bevorzugt in Form einer Lösung zum formiathaltigen Abwasser gegeben, deren Wasserstoffperoxidkonzentration im Bereich von etwa 9 bis 20 Mol/l (bis ca. 60 Gew-%) liegt. Für den Fall, dass die zugelassenen Transportvorschriften eingehalten werden, kann die Konzentration der eingesetzten Wasserstoffperoxidlösung bis auf über 90 Gew % erhöht werden. Bevorzugt wird das Wasserstoffperoxid im Überschuss zugegeben, wobei die zugegebene Menge bezogen auf das im Abwasser enthaltene Formiat im Bereich von 160 bis 200 Mol %, insbesondere bevorzugt 160 bis 170 Mol % gewählt wird. Neben Wasserstoffperoxid können auch andere Oxidationsmittel eingesetzt werden, beispielsweise Ozon oder Natriumhypochloritlösung. Bei der Auswahl der Oxidationsmittel spielen beispielsweise Gestehungskosten und Umweltgesetzgebung eine Rolle. In Abhängigkeit von den gesetzlichen Grenzwerten und den weiter zur Verfügung stehenden Reinigungsstufen ist es u.U. nicht erforderlich, die gesamte Menge der Formiationen zu oxidieren. Es kann bereits ausreichend sein, die Konzentration der Formiationen durch eine chemische oxidative Behandlung erheblich zu reduzieren und das so behandelte Abwasser ggf. beispielsweise einer biologischen Klärstufe zuzuführen.

Die Behandlung des formiathaltigen Abwassers mit Wasserstoffperoxid wird vorzugsweise bei 20 bis 95 °C, insbesondere bevorzugt bei 50 bis 80 °C in einem pH-Bereich von 4 - 8 insbesondere bevorzugt 5,0 bis 6,5 durchgeführt.

Gemäß einer Ausführungsform des erfindungsgemäßen Verfahrens erfolgt die oxidative Behandlung des formiathaltigen Abwassers bereits vor der Abtrennung des Niederschlags. Dazu kann nach dem Alterungsschritt beispielsweise eine geeignete Menge an Wasserstoffperoxid zur Suspension gegeben werden und der Niederschlag erst nach dem weitgehenden oxidativen Abbau der Formiationen abgetrennt werden. Alternativ kann im zeitlichen Verfahrensablauf die Zugabe der Wasserstoffperoxidlösung auch bereits zur Cu-, Zn-, Al-haltigen Formiatlösung erfolgen, wobei das Fällungsreagenz, Carbonationen, durch Oxidation aus Formiationen erzeugt wird.

Die oxidative Aufarbeitung des Abwassers durch Zugabe eines geeigneten Oxidationsmittels, wie Wasserstoffperoxid, ist apparativ recht einfach durchzuführen und ermöglicht auch die Behandlung von Abwässern, welche eine relativ hohe Konzentration an Formiationen aufweisen. Es ist aber auch möglich, dass die oxidative Behandlung allein durch eine biologische Behandlung des formiathaltigen Abwassers erfolgt. Gegebenenfalls kann das formiathaltige Abwasser dazu auf eine geeignete Konzentration verdünnt werden.

Die oxidative Behandlung des formiathaltigen Abwassers wird vorzugsweise in der Weise durchgeführt, dass die Formiatkonzentration im Abwasser nach der oxidativen Behandlung kleiner als 0,1 Gew.- % ist.

Vergleicht man den unter Verwendung der Formiate erhaltenen Cu/Zn/Al-Katalysator mit Katalysatoren, die ausgehend von den Nitraten hergestellt wurden, zeigt der mit dem erfindungsgemäßen Verfahren erhaltene Katalysator eine bessere bis vergleichbare Aktivität und eine vergleichbare Selektivität. Auch die bei 250 °C im Methanolsynthesetest bestimmte Langzeitstabilität des unter Verwendung der Formiate hergestellten Katalysators ist etwas besser als oder zumindest vergleichbar mit der Stabilität eines Katalysators, welcher ausgehend von den Metallnitraten hergestellt wurde.

Der erfindungsgemäße erhältliche Katalysator enthält in seiner oxidischen Form weniger als 5 Gew.- %, vorzugsweise zwischen 0,5 und 4 Gew.- %, insbesondere bevorzugt zwischen 1 und 2 Gew.- % Formiat, berechnet als Ameisensäure. Durch schonende Calcinierung kann die Formiatstruktur konserviert werden, die nach dem in der Katalyse häufig anzutreffenden Schlüssel-Schloss-Prinzip eine wichtige Rolle zum Erreichen einer hohen Aktivität spielen kann. Bei einer schonenden Calcinierung bei etwa 170 °C für 4 Minuten entsteht ein Katalysator, der bei 250 °C eine sehr hohe Aktivität zeigt.

Der erfindungsgemäß erhältliche Katalysator zeichnet sich durch ein hohes Mesoporenvolumen aus. Der Anteil der Mesoporen mit einem Radius von 3,75 bis 7,0 nm am Gesamtporenvolumen beträgt dabei vorzugsweise mehr als 30 %, vorzugsweise 30 bis 80 %. Das Gesamtporenvolumen umfasst das Volumen der Poren mit einem Radius von 3,75 bis 7.500 nm. Das Porenvolumen kann mit dem Quecksilberintrusionsverfahren bestimmt werden. Vorzugsweise beträgt das Gesamtporenvolumen, bestimmt an 6 x 4 mm Tabletten, 100 mm$^3$/g - 700 mm$^3$/g, vorzugsweise 250 mm$^3$/g - 450 mm$^3$/g.

Der Anteil des Kupfers, berechnet als CuO und bezogen auf das Gewicht der oxidischen Katalysatorform, wird unter Berücksichtigung des Glühverlustes bei 600 °C vorzugsweise zwischen 55 und 69 Gew.- %, insbesondere bevorzugt 60 und 63 Gew.- %, gewählt.

Der Anteil des Zinks, berechnet als ZnO und bezogen auf das Gewicht der oxidischen Katalysatorform, wird unter Berücksichtigung des Glühverlustes bei 600 °C vorzugsweise zwischen 20 und 33 Gew.- %, insbesondere bevorzugt zwischen 25 und 31 Gew.- %, gewählt.

Der Anteil des Aluminiums, berechnet als Al$_2$O$_3$ und bezogen auf das Gewicht der oxidischen Katalysatorform, wird vorzugsweise zwischen 5 und 20 Gew.- %, insbesondere bevorzugt zwischen 8 und 11 Gew.- %, gewählt.

Die Prozentangaben für die Anteile des Kupfers, Zinks und Aluminiums beziehen sich auf einen drei Stunden bei 600°C geglühten Katalysator.

Der erfindungsgemäß erhältliche Katalysator weist in der oxidischen Form weiterhin einen Alkaliionengehalt, insbesondere Natriumionengehalt, von bevorzugt weniger als 500 ppm, insbesondere bevorzugt weniger als 300 ppm, insbesondere 100 ppm bis 300 ppm auf.

In seiner oxidischen Form weist der erfindungsgemäß erhältliche Katalysator vorzugsweise eine spezifische Oberfläche von mehr als 90 $m^2$/g, insbesondere bevorzugt mehr als 100 $m^2$/g auf.

Der erfindungsgemäß erhältliche Katalysator kann an sich als Formkörper mit beliebiger Gestalt ausgeformt werden. Beispielsweise kann er in Form von Ringen, 3-20 Loch Formkörpern, Tabletten mit glatter oder gewellter Außenfläche oder Waben ausgestaltet sein. Die Dimensionierung der Formkörper entspricht den üblichen Werten. Für die Herstellung der Formkörper wird der pulverförmige Katalysator, ggf. unter Zusatz eines Gleitmittels, wie Graphit, verpresst, beispielsweise zu Tabletten von z.B. 6 x 4 mm.

Vor der Verwendung wird der Katalysator von der oxidischen Form in die aktive Form überführt. Dazu wird das Kupferoxid zumindest in einem Anteil zu elementarem Kupfer reduziert. Hierzu wird die oxidische Form des erfindungsgemäß erhältlichen Katalysators bevorzugt im Wasserstoffstrom reduziert. Die Aktivierung kann direkt im Synthesereaktor erfolgen und erfolgt vorzugsweise in der Weise, dass zunächst mit Hilfe eines eine kleine Menge Wasserstoff enthaltenden inerten Gases, wie Stickstoff, reduziert wird. Der Stickstoff enthält üblicherweise zunächst etwa 2,0 Vol.- % $H_2$. Hierbei wird die Temperatur beispielsweise von 100 auf 235 °C über einen Zeitraum von 16 Stunden angehoben. Danach wird der Wasserstoffanteil erhöht, wobei zum Beispiel mit 20 Vol.- % $H_2$ (Rest $N_2$) über einen Zeitraum von 3 Stunden im Temperaturintervall von 235 bis 270 °C reduziert wird. Die Vollendung der reduktiven Behandlung kann mit 99,9 %-igem $H_2$ über einen Zeitraum von etwa 3 Stunden bei 270 °C bis 300 °C erfolgen. Üblicherweise wird mit einer Raumgeschwindigkeit von etwa 3.000 bis 4.000 Liter Reduktionsgas pro Stunde und Liter Katalysator aktiviert.

Im reduzierten Zustand beträgt die Größe der Kupferkristallite vorzugsweise etwa 4 bis 12 nm, vorzugsweise 5 bis 7 nm.

Der erfindungsgemäß erhältliche Katalysator eignet sich insbesondere für eine Verwendung in der Methanolsynthese.

Die Synthese wird üblicherweise bei einer Temperatur von etwa 200 bis 320 °C, vorzugsweise bei 210 °C bis 280 °C, bei einem Druck von etwa 40 bis 150 bar, vorzugsweise bei etwa 60 bis 100 bar, und bei einer Raumgeschwindigkeit von etwa 2.000 bis 22.000, vorzugsweise von 8.000 bis 12.000 Liter Synthesegas pro Stunde und Liter Katalysator durchgeführt, wobei das Synthesegas etwa 5 bis 25, vorzugsweise 6 bis 12 Vol.- % CO, etwa 4 bis 10 Vol.- % $CO_2$, etwa 10 bis 30 Vol.- % $N_2$ plus $CH_4$ (inerte Gase) und als Rest $H_2$ enthalten kann.

Weiter eignet sich der erfindungsgemäß erhältliche Katalysator für die Verwendung in der Methanolreformierung sowie in der Tieftemperaturkonvertierung von Kohlenmonoxid zu Kohlendioxid. Letztere auch als Low-Temperature-Shift (LTS) bezeichnete Reaktion erfolgt bei Temperaturen im Bereich von etwa 175 bis 250 °C, bevorzugt 205 bis 215 °C und Steam/Gas-Verhältnissen im Bereich von etwa 0,4 bis 1,5 (Nl/Nl). Typische Feed-Gas-Mischungen enthalten etwa 3 Vol- % CO, 17 Vol.- % $CO_2$, 2 Vol- % $N_2$ und 78 Vol.- % $H_2$, die mit Raumgeschwindigkeiten von etwa 2.000 bis 12.000 l trockenem Gas (d.h. ohne Wasser) pro Liter Katalysator und Stunde durch den Reaktor geleitet werden. Gute Katalysatoren erreichen bei einer Raumgeschwindigkeit von 11.200 $h^{-1}$ und einem Verhältnis Dampf zu Gas von ungefähr 1,5 CO-Umsätze von 70 bis 85 %.

Die Erfindung wird im Weiteren anhand von Beispielen näher erläutert.

**BEISPIEL**

(a) Herstellung der Kupferlösung

5.054 g einer Suspension von Cu(OH)$_2$ x CuCO$_3$ (Cu-Gehalt: 27,7 Gew.- %, entsprechend 1.400 g Cu) werden in einem 10 1-Becherglas dispergiert und portionsweise mit insgesamt 2.399 ml 85 %-iger Ameisensäure (D = 1,1856 g/cm$_3$) versetzt, bis die $CO_2$-Entwicklung beendet ist. Die Kupferlösung weist einen pH von 2,35 und eine Temperatur von 55 °C auf.

(b) Herstellung der Cu/Zn-Lösung (Vorstufe erste Lösung)

In einem 5 1-Becherglas wird aus 781 g ZnO und 4000 ml $H_2O$ eine ZnO-Dispersion hergestellt. Die Suspension wird mit der unter (a) erhaltenen Kupferlösung vereinigt. Unter fortgesetztem Dispergieren werden weitere 890 ml Ameisensäure und 33,6 1 demineralisiertes Wasser portionsweise zugegeben. Die blaue, zunächst noch leicht opale Lösung wird solange gerührt, bis sie völlig klar ist.

(c) Herstellung der Natriumcarbonatlösung (zweite Lösung)

Es werden 24.000 ml $Na_2CO_3$-Lösung hergestellt, welche eine Konzentration von ca. 180 g $CO_3$/100 ml aufweist, und die Lösung auf 70 °C erwärmt.

(d) Herstellung der Aluminiumlösung I

In einem mit einer Kühleinrichtung versehenen 5 1-Rührwerk werden 2.193 ml entsalztes Wasser vorgelegt und 246,7 g $NaAlO_2$ hinzugefügt. Die Lösung wird auf eine Temperatur von maximal 30 °C temperiert und dann werden portionsweise 350 ml Ameisensäure zugegeben. Die milchige Lösung weist einen pH von ca. 4,0 auf.

(e) Herstellung der Aluminiumlösung II

In einem mit einer Kühleinrichtung versehenen Rührwerk werden 2.193 ml Wasser vorgelegt und 246,7 g $NaAlO_2$ portionsweise hinzugefügt. Dabei wird darauf geachtet, dass die Temperatur der Lösung 30 °C nicht überschreitet. Die Mischung wird solange gerührt, bis eine klare Lösung erhalten wird.

(f) Fällung

Die unter (b) erhaltene Lösung wird in einen ersten Vorratstank einer Mischapparatur überführt. Anschließend wird die unter (d) erhaltene Aluminiumlösung I in den Tank eingefüllt und dort solange bei Raumtemperatur gerührt, bis die vereinigten Lösungen völlig klar sind. Gegebenenfalls kann Wasser zugegeben werden, um die Opaleszenz der Lösung zu beseitigen. Vor Fällbeginn wird die Lösung auf 70 °C erwärmt. Ca. 30 Minuten vor Fällungsbeginn wird die unter (e) erhaltene Aluminiumlösung II in den Tank überführt, wobei sich eine milchige, weiß-bläuliche Suspension ausbildet.

In einen zweiten Vorratstank wird die unter (c) erhaltene $Na_2CO_3$-Lösung eingefüllt und auf 70 °C erwärmt.

Die im ersten und zweiten Tank enthaltenen Lösungen werden gleichzeitig einer Mischvorrichtung zugeführt und gelangen nach einer Verweilzeit von etwa 20 s von dort in einen Überlaufbehälter. Die Pumpraten werden so eingestellt, dass der pH während der Fällung etwa $6,5 \pm 0,1$ beträgt. Von der Mischvorrichtung gelangt die Mischung in einen Überlaufbehälter. Vom Überlaufbehälter gelangt die entstandene Suspension in einen Alterungsbehälter, wo sie unter Rühren bei ca. 65 °C gehalten wird. Die Fällung ist nach ca. 35 Minuten beendet. Anschließend wird die Anlage mit ca. 600 ml entsalztem Wasser gespült und die Temperatur im Alterungsbehälter auf 70 °C erhöht. Nach Beendigung des Spülvorgangs wird die Suspension unter Rühren gealtert. Der Beginn der Alterung wird durch das Ende des Spülvorgangs definiert. Die in den Beispielen angewandten Alterungszeiten sind in Tabelle 2 genannt.

Nach Beendigung des Alterns wird die Suspension filtriert, mit entsalztem Wasser gewaschen, bis der Restgehalt an Natrium im Filterkuchen auf weniger als 350 ppm gefallen ist und der erhaltene Feststoff durch Sprühtrocknung unter Verwendung einer Einstoffdüse im Gegenstrom getrocknet. Als Zulauf wird eine Suspension mit einem Trockensubstanzgehalt von 30 Gew.- % eingestellt. Die Heizgaseintrittstemperatur beträgt 330 - 350 °C, die Produktaustrittstemperatur 110 - 120 °C. Das getrocknete Pulver wird anschließend entweder in Porzellanschalen in einem Hordenofen oder in einem ansatzweise betriebenen Labor-Drehrohrofen bei etwa 320 °C 50 Minuten calciniert.

(g) Behandlung des Abwassers

Das bei der Filtration und dem Waschen anfallende Abwasser wird durch Zugabe von Wasserstoffperoxid behandelt, wobei der pH mit Schwefelsäure zwischen 5 und 6,5 und die Temperatur des Abwassers bei ca. 70 °C gehalten werden. Die Formiatkonzentration des Abwassers wird dabei auf Werte zwischen 2,8 Gew.- % und 0,09 Gew.-% eingestellt. Die Selektivität, definiert als Mol umgesetztes Formiat/Mol eingesetztes Wasserstoffperoxid, beträgt ca. 60 %.

**VERGLEICHSBEISPIEL**

(a) Herstellung einer Kupfer/Zink-Nitratlösung

Zu 9,79 kg einer Kupfernitratlösung, welche 1400 g Kupfer enthält, werden 781,25 g Zinkoxid gegeben. Dann werden 2077 g Salpetersäure (58 %) zugegeben und die Mischung solange gerührt, bis sich die Feststoffe vollständig aufgelöst haben.

(b) Herstellung einer Aluminiumnitratlösung

In 1,5 l vollentsalztem Wasser werden 246,7 g $Na_2AlO_2$ gelöst. Dann werden 1365 g Salpetersäure (58 %) zugegeben und solange gerührt, bis eine klare Lösung erhalten wird. Die erhaltene Aluminiumnitratlösung wird zur Kupfer/Zink-Nitratlösung gegeben und die Cu/Zn/Al-Lösung auf 60 °C erwärmt.

(c) Herstellung des Aluminiumsols

In 1,5 l vollentsalztem Wasser werden 246,7 g $Na_2AlO_2$ während 30 Minuten unter Rühren gelöst. Die erhaltene Lösung wird zur Cu/Zn/Al-Lösung gegeben und die Mischung auf 60 °C erwärmt.

(d) Fällung

In den ersten Vorratstank einer Mischungsapparatur wird die nach (c) erhaltene Mischung eingefüllt. In den zweiten Vorratstank der Mischungsapparatur werden 25 l einer wässrigen Lösung eingefüllt, welche 172 g $Na_2CO_3$ pro Liter enthält. Die beiden Lösungen werden gleichzeitig in einen Mischungsbehälter gepumpt und die Mischung von dort in einen Alterungsbehälter geleitet.

Nach Fällungsende wird der Mischungsbehälter mit vollentsalztem Wasser gespült, die Temperatur im Alterungsbehälter auf 70 °C erhöht und der Niederschlag für 1 bzw. 4 Stunden gealtert. Nach Beendigung des Alterns wird die Suspension filtriert, mit entsalztem Wasser gewaschen, bis der Restgehalt an Natrium im Filterkuchen auf weniger als 350 ppm gefallen ist und der erhaltene Feststoff durch Sprühtrocknung unter Verwendung einer Einstoffdüse im Gegenstrom getrocknet. Die Heizgaseintrittstemperatur beträgt 330 - 350 °C, die Produktaustrittstemperatur 110 - 120 °C. Das getrocknete Pulver wird anschließend entweder in Porzellanschalen in einem Hordenofen oder in einem ansatzweise betriebenen Labor-Drehrohrofen bei etwa 320 °C 50 Minuten calciniert.

Die durchgeführten Fällungsbeispiele sowie die nachfolgend angewandten Calcinierbedingungen sind in Tabellen 2a + b zusammengefasst. Ebenfalls sind in diesen Tabellen die chemischen Zusammensetzungen und die physikalischen Parameter der erhaltenen oxidischen Katalysatorvorstufen mit aufgenommen.

Die Bestimmung der physikalischen Parameter wird auf die folgende Weise vorgenommen:

Bestimmung der Cu-Kristallitgröße:

Die Größe der Cu-Kristallite wird mittels Röntgen-Pulverdiffraktometrie (XRD) bestimmt. Der Cu (111) Reflex im Bereich von - 43,3°(2θ) wird vermessen. Die Halbwertsbreite und die integrale Intensität des Reflexes wird mit der Pseudo-Voigt-Funktion berechnet. Die Cu-Kristallitgröße wird mit Hilfe der Scherrer-Funktion auf der Basis der berechneten Halbwertsbreite berechnet.

Zur Vorbereitung auf die röntgenographische Bestimmung der Kristallitgröße werden die oxidischen Katalysatoren wie folgt reduziert:

2 - 5 g Tabletten mit einer Größe von 6 x 4 mm werden in einem Röhrenreaktor mit einem Reduktionsgas (98 % $N_2$, 2 % $H_2$) mit einer Heizrate von 2 °C/min von Raumtemperatur auf eine Maximaltemperatur erwärmt. Katalysatoren, welche aus den Formiaten hergestellt wurden, werden auf eine Maximaltemperatur von 80 - 120 °C erhitzt. Katalysatoren, welche unter Verwendung der Nitrate hergestellt wurden, werden zunächst über Nacht auf 175 °C erhitzt. Ansdhließend wird innerhalb von weniger als 2 Stunden die Maximaltemperatur eingestellt, für Katalysatoren aus a) der Formiat-/Carbonat-Route 180 °C; b) der Nitrat-/Carbonat-Route 240 °C. Unter Beibehaltung der Maximaltemperatur wird schließlich der Wasserstoffgehalt des Reduktionsgases innerhalb einer Stunde auf 100 % erhöht und dann die Probe für weitere 3 Stunden reduziert.

Bestimmung der spezifischen Oberfläche

Die BET-Oberfläche wird nach der Stickstoff-Einpunktmethode am pulverförmigen oxidischen Katalysator sowie an 6 x 4 mm Tabletten in Anlehnung an DIN 66132 bestimmt.

Bestimmung des Glühverlustes

Sofern der Glühverlust an Tabletten bestimmt werden soll, werden diese zunächst zu einem Pulver gemahlen. Die zu bestimmende Probe wird in einem gewogenen Porzellantiegel eingewogen, der zuvor 3 Stunden bei 600 °C in einem Muffelofen erhitzt und dann im Exsikkator auf Raumtemperatur abgekühlt worden ist. Der Tiegel wird für 3 Stunden in einem Muffelofen auf 600 °C erhitzt und anschließend im Exsikkator auf Raumtemperatur abgekühlt. Der abgekühlte Tiegel wird erneut gewogen und aus der Differenz der Glühverlust bei 600°C bestimmt.

Bestimmung der Seitendruckfestigkeit

Die Seitendruckfestigkeit wird nach DIN EN 1094-5, Ausgabe 1995-09, feuerfeste Erzeugnisse für Isolationszwecke - Teil 5: Bestimmung der Kaltdruckfestigkeit geformter Erzeugnisse bestimmt. Die Bestimmung wird mit einem handelsüblichen Gerät wie Schleuninger 6-D oder ERWEKA TBH 310 MD nach den Angaben des Geräteherstellers durchgeführt.

Für eine repräsentative Probenmenge von 100 Tabletten werden die an deren Zylindermantel anliegende Pressdrücke beim Berstvorgang bestimmt und mit dem geräteeigenen Statistikprogramm bezüglich Mittelwert, Standardabweichung sowie Minimal- und Maximalhärte ausgewertet. Die Verteilung der Tablettenhärte (N) wird grafisch dargestellt.

Bestimmung des Porenvolumens

Das Porenvolumen wird nach der Quecksilber-Intrusionsmethode in Anlehnung an DIN 66133 an den pulverförmigen oxidischen Katalysatoren sowie an 6 x 4 mm Tabletten bestimmt.

Bestimmung des Formiatgehalts

Ca. 10 bis 20 g des calcinierten Katalysatorpulvers werden in einem 300 ml Erlenmeyerkolben in 25 + x ml $H_2SO_4$ (25 %) aufgenommen und unter Erwärmen auf ca. 70 °C gelöst. Die Menge x ml $H_2SO_4$ (25 %) ist bestimmt durch die minimale ggf. zusätzlich erforderliche Schwefelsäuremenge um vollständiges Auflösen der Einwaage zu erreichen. Es wird mit destilliertem Wasser auf ca. 100 ml aufgefüllt. Durch Zugabe von ca. 2,5 bis 25 ml NaOH (30 %) wird die Lösung auf einen pH-Wert von 8 bis 10 eingestellt. Die Lösung wird dann für mindestens weitere 5 Minuten auf 70 °C erwärmt. Es werden anschließend 20 ml $KMnO_4$-Lösung (0,2 N) zugegeben und die Lösung für mindestens 30 Min. zum leichten Sieden erhitzt. Die heiße Lösung wird mit $H_2SO_4$ (25 - 50 ml) angesäuert und 20 ml Oxalsäure (0,2 N) zugegeben, um Mangandioxid und überschüssiges KMnO4 zu Mn2+ zu reduzieren. Die zugesetzte Oxalsäure-Lösung muss im Gehalt an Reduktionsäquivalenten exakt den Oxidationsäquivalenten der Permanganatlösung entsprechen. Die klare Lösung wird letztendlich mit 0,2 N $KMnO_4$ titriert, bis eine leichte Rosafärbung beobachtet wird.

Berechnung:

Jeder ml verbrauchter 0,2 N $KMnO_4$-Lösung entspricht 4,5 mg Formiat. Der prozentuale Gehalt der Probe an Formiat ergibt sich zu:

```
[ % Formiat] = [(ml verbrauchte 0,2 N KMnO₄-Lsg) x (4,5 mg For-
miat/ml 0,2 N KMnO₄-Lsg ) x 100)/ (Einwaage Probenmenge mg]
```

Die Methode liefert abgesicherte Ergebnisse für Formiatkonzentrationen in der zu titrierenden Lösung zwischen 0,08 und 0,5 Gew-% mit Abweichungen < +8%. Diese Abweichungen können bei Verwendung einer 0,02 N-Permanganatlösung reduziert werden auf +/-2%. Wegen der Abhängigkeit der Methodengenauigkeit von der an sich unbekannten Formiatkonzentration kann es erforderlich sein, die Einwaage zunächst an einem erwarteten Wert auszurichten um nach Vorliegen des ersten Ergebnisses die Titration mit einer angepassten Einwaage zu wiederholen, die dann für die zu titrierende Lösung Formiatkonzentrationen im oben genannten Bereich ergibt.

Die Formiatgehalte der Proben aus den Beispielen 3 und 5 sind in Tabelle 1 angegeben

Tabelle 1: Formiatgehalt calcinierter Katalysatoren (Werte t.q.= nicht auf GV bezogen)

| Beispiel | Formiatgehalt |
|---|---|
| 3 | 1,3 % |
| 5 | 1,1 % |

Die physikalischen Eigenschaften der hergestellten Katalysatoren sind in Tabelle 2 wiedergegeben. Tabelle 2a bezieht sich dabei auf die pulverförmigen Katalysatoren, während sich die Werte aus Tabelle 2b auf Katalysatoren beziehen, welche zu Tabletten mit den Abmessungen 6 x 4 mm verpresst wurden.

**Tabelle 2a physikalische Daten der hergestellten oxidischen Katalysatorpulver**

| Nr. | Alterung (h) | Calcinierung Hordenofen, 5 mm Schütthöhe (°C/min) | Cu (%) | Zn(%) | Al (%) | spez. O-berfl.[3] $(m^2/g)$ | KGV[4] $D_{50}$ $(\mu m)$ | GV (600 °C) | Prozess[7] |
|---|---|---|---|---|---|---|---|---|---|
| 0[1] | | | 48,3 | 22,1 | 4,9 | | | 9,4 | N |
| 1[2] | 1 | 320/50 | 50,1 | 21,5 | 5,3 | 126,0 | 23,3 | 6,9 | N |
| 2[2] | 4 | 320/50 | 50,1 | 21,5 | 5,3 | 117,0 | 21,2 | 4,7 | N |
| 3[2] | 1 | 320/50 | 49,6 | 21,8 | 5,1 | 134,0 | 20,3 | 15,2 | F |
| 4[2] | 1 | 350/100 | 49,6 | 21,8 | 5,1 | 129,0 | 20,2 | 6,8 | F |
| 5[2] | 4 | 320/50 | 49,2 | 22,4 | 5,0 | 130,0 | 19,4 | 14,7 | F |
| 6[2] | 4 | 350/100 | 49,2 | 22,4 | 5,0 | 123,0 | 18,8 | 6,4 | F |
| 7[8] | 1 | 320/105 | 49,5 | 18,6 | 5,0 | 102,0 | 15,6 | 7,2 | F |
| 8[8] | 4 | 320/105 | 49,5 | 18,6 | 5,0 | 113,0 | 14,7 | 8,2 | F |
| [1]: Industrie-Standard [2]: hergestellt unter Verwendung eines Mischrohrs Typ A [3]: BET [4]: Korngrößenverteilung [7]: N = Nitratweg; F = Formiatweg [8]: hergestellt unter Verwendung eines Mischrohrs Typ B | | | | | | | | | |

**Tabelle 2b: physikalische Daten der aus den Katalysatoren hergestellten Tabletten**

| Nr. | spez. Oberfl.[3] $(m^2/g)$ | SDF (N)[5] | Größe Cu-Kristallite (Å) | PV[6] R = 3,75 - 7500 nm $(mm^3/g)$ | PV[7] R = 3,75 - 7,0 nm $(mm^3/g)$ | PV[7]/ PV[6] (%) | PV[7] (Probe)/ (Standard) % |
|---|---|---|---|---|---|---|---|
| 0 | 80,3 | 155,0 | 75,0 | 268, 8 | 82,8 | 30,8 | 100 |
| 1 | 93,0 | 159,8 | 67,8 | 256,7 | 128,3 | 50,0 | 155,0 |
| 2 | 99,0 | 157,0 | 63,4 | 283,9 | 136,0 | 47,9 | 164,3 |
| 3 | 115,0 | 157,9 | 62,7 | 425,2 | 159,0 | 37,4 | 192,1 |
| 4 | 104,0 | 176,3 | 50,9 | 340,2 | 140,6 | 41,3 | 169,9 |
| 5 | 111,0 | 159,1 | 59,9 | 435,8 | 149,5 | 34,3 | 180,6 |
| 6 | 99,0 | 185,1 | 56,6 | 281,1 | 150,8 | 53,6 | 182,2 |
| 7 | 91,0 | 213,9 | 67,3 | 277,1 | 92,5 | 33,4 | 111,8 |
| 8 | 99,0 | 202,7 | 55,4 | 286,7 | 102,9 | 35,9 | 124,3 |
| [5]: Seitendruckfestigkeit [6,7] : Porenvolumen bestimmt nach dem Hg-Intrusionsverfahren bei 2000 bar | | | | | | | |

Methanolaktivitätstests

Die oxidierten Tabletten werden geviertelt, eine Siebfraktion von 2,5 bis 3,5 mm in einen Rohrreaktor gefüllt und nach Aktivierung einem standardisierten Aktivitätstest mit periodisch wechselnden Temperaturen unterworfen. In der Auswertung werden die Gewichts-Zeit-Ausbeuten (GZA) in kg(Methanol)/(kg(Katalysator) x h) als Mittelwert für je eine Periode konstanter Temperatur ermittelt. In einem Testreaktor-System bestehend aus 6 - 16 Einzelrohren wird in einem Testrohr als Standard der Katalysator C79-7 der Firma Süd-Chemie AG, DE, München eingesetzt und die für die anderen Proben bestimmte GZA jeweils ins Verhältnis zum Wert des Standards gesetzt. Dieses Verfahren hat den Vorteil, dass geringe Schwankungen z.B. in der Synthesegaszusammensetzung für alle Muster gleich sind und somit die Ergebnisse aus verschiedenen Testläufen miteinander verglichen werden können.

Nebenprodukte werden mit Hilfe gaschromatografischer Untersuchung von Kondensatproben, denen jeweils ein interner Standard zugesetzt ist, bestimmt. Die ermittelten Werte werden ebenfalls relativ zu den Werten angegeben, die für den Standard erhalten werden.

Die Ergebnisse des Methanolaktivitätstests sind in Tabelle 3 aufgeführt.

**Tabelle 3: relative Gewicht-Zeit-Ausbeuten bezogen auf Standardkatalysator**

| Nr. | GZA | | | | Nebenprodukte gesamt | | | | Nebenprodukt Ethanol | | | |
|---|---|---|---|---|---|---|---|---|---|---|---|---|
| | 250°C | 230°C | 210°C | 250°C | 250°C | 230°C | 210°C | 250°C | 250°C | 230°C | 210°C | 250°C |
| 0 | 100,0[1] | 100,0[2] | 100,0[3] | 100,0[4] | 100,0[1] | 100,0[2] | 100,0[3] | 100,0[4] | 100,0[1] | 100,0[2] | 100,0[3] | 100,0[4] |
| 1 | 92,2[1] | 86,2[2] | 82,6[3] | 93,0[4] | 89[1] | 86[2] | 87[3] | 108[4] | 92[1] | 97[2] | 99[3] | 98[4] |
| 2 | 96,0[1] | 94,2[2] | 93,5[3] | 97,6[4] | 97[1] | 93[2] | 86[3] | 111[4] | 96[1] | 91[2] | 68[3] | 104[4] |
| 3 | 103[5] | 96[6] | 91[7] | 99[8] | 99[5] | 95[6] | 115[7] | 97[8] | 98[5] | 96[6] | 163[7] | 93[8] |
| 4 | 106[5] | 102[6] | 96' | 102[8] | 101[5] | 104[6] | 120[7] | 109[8] | 111[5] | 118[6] | 235[7] | 109[8] |
| 5 | 102,4[1] | 96,9[2] | 97,1[3] | 101,0[4] | 103[1] | 89[2] | 95[3] | 114[4] | 102[1] | 89[2] | 61[3] | 99[4] |
| 6 | 99, 2[1] | 99,0[2] | 100,9[3] | 99,1[4] | 113[1] | 92[2] | 78[3] | 109[4] | 107[1] | 72[2] | 34[3] | 94[4] |
| 7 | 96,4[9] | | 78,9[10] | 92,2[11] | 102[9] | | 111[10] | 109[11] | 103[9] | | 194[10] | 107[11] |
| 8 | 103,5[9] | | 100,5[10] | 102,0[11] | 109[9] | | 98[10] | 117[11] | 119[9] | | 128[10] | 119[11] |

**Tabelle 3b: Versuchsdauer (time on stream "TOS")**

| Index | Dauer (h) | Index | Dauer (h) | Index | Dauer (h) | Index | Dauer (h) |
|---|---|---|---|---|---|---|---|
| 1 | 0 - 96 | 2 | 96 - 144 | 3 | 144 - 192 | 4 | 192 - 240 |
| 5 | 0 - 75 | 6 | 75 - 171 | 7 | 171 - 243 | 8 | 243 - 339 |
| 9 | 0-96 | 10 | 96 - 186 | 11 | 186 - 231 | | |

**Patentansprüche**

1.  Verfahren zur Herstellung von Cu/Zn/Al-Katalysatoren, **dadurch gekennzeichnet, dass**

    - eine erste wässrige Lösung hergestellt wird, welche zumindest Kupferformiat und Zinkformiat enthält, indem

        • eine wässrige Kupferformiatlösung hergestellt wird, indem ein Kupfersalz durch Zugabe von Ameisensäure rückstandsfrei gelöst wird,
        • eine wässrige Dispersion oder Lösung eines Zinksalzes hergestellt wird,
        wobei bezogen auf die eingesetzte Menge an Kupfer- und Zinksalz unter Berücksichtigung der Stöchiometrie die Ameisensäure in einem Überschuss von mindestens 10 Mol-% vorliegt,
        • eine wässrige Aluminiumsalzlösung hergestellt wird, die Lösung oder Dispersion des Zinksalzes mit der Kupferformiatlösung zu einer Kupfer/Zink-Lösung vereinigt wird, wobei die erhaltene Kupfer/Zink-Lösung einen pH im Bereich von 3,0 bis 4,0 aufweist, und die Aluminiumsalzlösung zur Kupfer/Zink-Lösung gegeben wird,
        wobei die Aluminiumsalzlösung in mehreren Teillösungen zugegeben wird, wobei zumindest ein erster Anteil der Aluminiumsalzlösung hergestellt wird, indem zumindest ein erster Teil des Aluminiumsalzes unter Zugabe von Ameisensäure in Wasser gelöst wird bis der pH-Wert im Bereich von weniger als 5 liegt, und ein zweiter Anteil der Aluminiumsalzlösung hergestellt wird, indem ein zweiter Anteil des Aluminiumsalzes in Wasser gelöst wird, so dass der pH-Wert der erhaltenen $NaAlO_2$-Lösung im Bereich von 11 bis 14 liegt, und zur Herstellung der ersten Lösung der erste Anteil der Aluminiumsalzlösung und der zweite Anteil der Aluminium-salzlösung zeitlich versetzt zu der Kupfer/Zink-Lösung gegeben werden,
        so dass die erste Lösung ein Aluminiumhydroxidsol/gel-Gemisch enthält,

    - eine zweiten Lösung hergestellt wird, welche ein Fällreagenz enthält,
    - in einem Fällungsschritt die erste Lösung und die zweite Lösung vereinigt werden, wobei ein Niederschlag erhalten wird,
    - der Niederschlag von der ein Abwasser bildende wässrigen Phase abgetrennt wird, und
    - der Niederschlag gewaschen wird, bis ein Alkaligehalt, bezogen auf den bei 600 °C geglühten Katalysator, von weniger als 500 ppm erreicht ist und
    - der Niederschlag anschließend getrocknet wird.

2.  Verfahren nach Anspruch 1, wobei die Kupferformiatlösung einen pH-Wert von weniger als 3, vorzugsweise weniger als 2,5 aufweist.

3.  Verfahren nach Anspruch 1 oder 2, wobei die Kupfer/Zink-Lösung einen pH im Bereich von 3,5 bis 3,7 aufweist.

4.  Verfahren nach einem der Ansprüche 1 bis 3, **dadurch gekennzeichnet, dass** die wässrige Aluminiumsalzlösung, der erste Anteil der Aluminiumsalzlösung und/oder der zweite

    - Anteil der Aluminiumsalzlösung vor dem Fällen auf Temperaturen von maximal 40 °C, insbesondere maximal 30 °C, erwärmt wird.

5.  Verfahren nach einem der vorhergehenden Ansprüche, wobei während des Fällungsschritts ein pH im Bereich von 3,5 bis 7,5, vorzugsweise 6,0 bis 7,0 insbesondere bevorzugt 6,5 ± 0,1, eingehalten wird.

6.  Verfahren nach einem der vorhergehenden Ansprüche, **dadurch gekennzeichnet, dass** das Kupfersalz ausgewählt ist aus CuO, $Cu(OH)_2$, und $Cu(OH)_2$ x $CuCO_3$.

7. Verfahren nach einem der vorherigen Ansprüche, **dadurch gekennzeichnet, dass** als Zinksalz ZnO gewählt wird.

8. Verfahren nach einem der vorhergehenden Ansprüche, wobei das Fällreagenz eine Alkalibase, vorzugsweise ein Alkalicarbonat ist.

9. Verfahren nach einem der Ansprüche 1 bis 7, wobei das Fällreagenz Wasserstoffperoxid ist.

10. Verfahren nach einem der vorhergehenden Ansprüche, **dadurch gekennzeichnet, dass** der Niederschlag nach der Fällung gealtert wird.

11. Verfahren nach Anspruch 10, **dadurch gekennzeichnet, dass** die Alterung während einer Dauer von 10 Minuten bis 10 Stunden, vorzugsweise 1 bis 5 Stunden, erfolgt.

12. Verfahren nach Anspruch 10 oder 11, **dadurch gekennzeichnet, dass** die Alterung bei einer Temperatur von mehr als 60 °C, insbesondere im Bereich von 65 bis 80 °C, erfolgt.

13. Verfahren nach einem der vorhergehenden Ansprüche, **dadurch gekennzeichnet, dass** der Fällungsschritt als kontinuierliche Fällung durchgeführt wird.

14. Verfahren nach einem der vorhergehenden Ansprüche, **dadurch gekennzeichnet, dass** die erste Lösung Kupfer und Zink in einem Verhältnis enthält, das zwischen 1 : 99 und 99 : 1 gewählt ist.

15. Verfahren nach einem der vorhergehenden Ansprüche, **dadurch gekennzeichnet, dass** der Niederschlag nach dem Trocknen calciniert wird.

16. Verfahren nach Anspruch 15, wobei der Niederschlag calciniert wird bei Temperaturen im Bereich von 140 - 1000 °C, vorzugsweise 170 - 350 °C für eine Dauer von mindestens 0,1 Sekunden, vorzugsweise 20 Minuten bis 8 Stunden, insbesondere bevorzugt 30 Minuten bis 4 Stunden -.

17. Verfahren nach einem der vorhergehenden Ansprüche, **dadurch gekennzeichnet, dass** das Abwasser Formiationen enthält und das formiathaltige Abwasser einer oxidativen Behandlung unterworfen wird, wobei die Formiationen im Wesentlichen zu Carbonat, Hydrogencarbonat, Kohlendioxid und Wasser oxidiert werden.

18. Verfahren nach Anspruch 17, **dadurch gekennzeichnet, dass** zur oxidativen Behandlung Wasserstoffperoxid zum formiathaltigen Abwasser gegeben wird.

19. Verfahren nach Anspruch 17 oder 18, **dadurch gekennzeichnet, dass** die oxidative Behandlung des formiathaltigen Abwassers vor der Abtrennung des Niederschlags erfolgt.

20. Verfahren nach einem der Ansprüche 17 bis 19, **dadurch gekennzeichnet, dass** die oxidative Behandlung durch eine biologische Behandlung des formiathaltigen Abwassers erfolgt.

**Claims**

1. A process for preparing Cu/Zn/Al catalysts, **characterized in that**

   - a first aqueous solution which comprises at least copper formate and zinc formate is prepared by

     • preparing an aqueous copper formate solution by dissolving a copper salt without residue by adding formic acid,
     • preparing an aqueous dispersion or solution of a zinc salt, wherein, based on the amount of copper salt and zinc salt used, taking account of the stoichiometry, the formic acid is present in an excess of at least 10 mol%,
     • preparing an aqueous aluminum salt solution,
     combining the solution or dispersion of the zinc salt with the copper formate solution to give a copper/zinc solution, the resulting copper/zinc solution having a pH in the range from 3.0 to 4.0, and
     adding the aluminum salt solution to the copper/zinc solution,

wherein the aluminum salt solution is added in several part-solutions, wherein at least a first portion of the aluminum salt solution being prepared by dissolving at least a first portion of the aluminum salt in water with addition of formic acid until the pH is in the range of less than 5, and a second portion of the aluminum salt solution is

prepared by dissolving a second portion of the aluminum salt in water such that the pH of the $NaAlO_2$ solution obtained is in the range from 11 to 14, and wherein, to prepare the first solution, the first portion of the aluminum salt solution and the second portion of the aluminum salt solution are added offset in time to the copper/zinc solution, so that the first solution comprises an aluminum hydroxide sol/gel mixture,

- a second solution which comprises a precipitation reagent is prepared,
- in a precipitation step, the first solution and the second solution are combined to obtain a precipitate,
- the precipitate is removed from the aqueous phase, which forms wastewater, and
- the precipitate is washed until an alkali metal content, based on the catalyst calcined at 600°C, of less than 500 ppm is attained, and
- the precipitate is then dried.

**2.** The process as claimed in claim 1, wherein the copper formate solution has a pH of less than 3, preferably less than 2.5.

**3.** The process as claimed in claim 1 or claim 2, wherein the copper/zinc solution has a pH in the range from 3.5 to 3.7.

**4.** The process as claimed in one of claims 1 to 3, **characterized in that** the aqueous aluminum salt solution, the first portion of the aluminum salt solution and/or the second portion of the aluminum salt solution, before the precipitation, is/are heated to temperatures of not more than 40°C, especially not more than 30°C.

**5.** The process as claimed in one of the preceding claims, wherein, during the precipitation step, a pH in the range from 3.5 to 7.5, preferably from 6.0 to 7.0, especially preferably of $6.5 \pm 0.1$, is maintained.

**6.** The process as claimed in one of the preceding claims, **characterized in that** the copper salt is selected from CuO, $Cu(OH)_2$ and $Cu(OH)_2 \cdot CuCO_3$.

**7.** The process as claimed in one of the preceding claims, **characterized in that** the zinc salt selected is ZnO.

**8.** The process as claimed in one of the preceding claims, wherein the precipitation reagent is an alkali metal base, preferably an alkali metal carbonate.

**9.** The process as claimed in one of claims 1 to 7, wherein the precipitation reagent is hydrogen peroxide.

**10.** The process as claimed in one of the preceding claims, **characterized in that** the precipitate is aged after the precipitation.

**11.** The process as claimed in claim 10, **characterized in that** the aging is effected over a period of from 10 minutes to 10 hours, preferably from 1 to 5 hours.

**12.** The process as claimed in claim 10 or 11, **characterized in that** the aging is effected at a temperature of more than 60°C, especially in the range from 65 to 80°C.

**13.** The process as claimed in one of the preceding claims, **characterized in that** the precipitation step is performed as a continuous precipitation.

**14.** The process as claimed in one of the preceding claims, **characterized in that** the first solution comprises copper and zinc in a ratio which is selected between 1:99 and 99:1.

**15.** The process as claimed in one of the preceding claims, **characterized in that** the precipitate is calcined after drying.

**16.** The process as claimed in claim 15, wherein the precipitate is calcined at temperatures in the range of 140 - 1000°C, preferably 170 - 350°C, for a period of at least 0.1 second, preferably from 20 minutes to 8 hours, especially preferably from 30 minutes to 4 hours.

**17.** The process as claimed in one of the preceding claims, **characterized in that** the wastewater comprises formate ions and the formate-containing wastewater is subjected to an oxidative treatment, which oxidizes the formate ions essentially to carbonate, hydrogen carbonate, carbon dioxide and water.

**18.** The process as claimed in claim 17, **characterized in that** hydrogen peroxide is added to the formate-containing wastewater for the oxidative treatment.

**19.** The process as claimed in claim 17 or 18, **characterized in that** the oxidative treatment of the formate-containing wastewater is effected before the removal of the precipitate.

**20.** The process as claimed in one of claims 17 to 19, **characterized in that** the oxidative treatment is effected by a biological treatment of the formate-containing wastewater.

**Revendications**

**1.** Procédé pour la production de catalyseurs Cu/Zn/Al, **caractérisé**

- **en ce qu'**une première solution aqueuse qui contient au moins du formiate de cuivre et du formiate de zinc est produite, en

• produisant une solution de formiate de cuivre aqueuse par dissolution d'un sel de cuivre par ajout d'acide formique sans résidu,
• une dispersion ou solution aqueuse d'un sel de zinc est produite, dans laquelle l'acide formique rapporté à la quantité utilisée de sel de cuivre et de zinc est présent en un excédent d'au moins 10 % molaire compte tenu de la stoechiométrie,
• une solution de sel d'aluminium aqueuse est produite, la solution ou dispersion du sel de zinc est combinée avec la solution de formiate de cuivre pour produire une solution de cuivre / zinc, la solution de cuivre / zinc obtenue présentant un pH compris dans une plage allant de 3,0 à 4,0 et la solution de sel d'aluminium est ajoutée à la solution cuivre / zinc, la solution de sel d'aluminium étant ajoutée en plusieurs solutions partielles, moyennant quoi au moins une première portion de solution de sel d'aluminium est produite, en dissolvant au moins une première portion de sel d'aluminium dans l'eau par ajout d'acide formique jusqu'à l'obtention d'un pH s'inscrivant dans une plage inférieure à 5, et une deuxième portion de solution de sel d'aluminium est produite, en dissolvant une deuxième portion de sel d'aluminium dans l'eau de sorte que le pH de la solution $NaAlO_2$ obtenue s'inscrive dans une plage allant de 11 à 14 et en vue de la production de la première solution, la première portion de la solution de sel d'aluminium et la deuxième portion de la solution de sel d'aluminium sont ajoutées de manière différée dans le temps à la solution de cuivre / zinc, de sorte que la première solution contienne un mélange de gel / solution d'hydroxyde d'aluminium,

- **en ce qu'**une deuxième solution qui contient un agent précipitant est produite,
- dans une étape de précipitation, la première solution et la deuxième solution sont combinées, moyennant quoi un précipité est obtenu,
- le précipité est séparé de la phase aqueuse formant une eau résiduelle et
- le précipité est lavé jusqu'à l'obtention d'une teneur alcaline inférieure à 500 ppm, rapportée au catalyseur calciné à 600 °C et
- le précipité est ensuite séché.

**2.** Procédé selon la revendication 1, dans lequel la solution de formiate de cuivre présente un pH inférieur à 3, de préférence inférieur à 2,5.

**3.** Procédé selon la revendication 1 ou 2, dans lequel la solution de cuivre / zinc présente un pH s'inscrivant dans une plage allant de 3,5 à 3,7.

**4.** Procédé selon l'une quelconque des revendications 1 à 3, **caractérisé en ce que** la solution de sel d'aluminium aqueuse, la première portion de la solution de sel d'aluminium et / ou la deuxième portion de sel d'aluminium est chauffée avant la précipitation à une température de 40 °C maximum, en particulier de 30 °C maximum.

**5.** Procédé selon l'une quelconque des revendications précédentes, dans lequel, durant l'étape de précipitation, un pH s'inscrivant dans une plage allant de 3,5 à 7,5, de préférence de 6,0 à 7,0, de manière particulièrement préférée de 6,5 à ± 0,1 est respecté.

6. Procédé selon l'une quelconque des revendications précédentes, **caractérisé en ce que** le sel de cuivre est sélectionné parmi CuO, $Cu(OH)_2$, et $Cu(OH)_2.CuCO_3$.

7. Procédé selon l'une quelconque des revendications précédentes, **caractérisé en ce que** ZnO est sélectionné en tant que sel de zinc.

8. Procédé selon l'une quelconque des revendications précédentes, dans lequel l'agent précipitant est une base alcaline, de préférence un carbonate alcalin.

9. Procédé selon l'une quelconque des revendications 1 à 7, dans lequel l'agent précipitant est le peroxyde d'hydrogène.

10. Procédé selon l'une quelconque des revendications précédentes, **caractérisé en ce que** le précipité est vieilli après la précipitation.

11. Procédé selon la revendication 10, **caractérisé en ce que** le vieillissement se fait durant une période de 10 minutes à 10 heures, de préférence de 1 à 5 heures.

12. Procédé selon la revendication 10 ou 11, **caractérisé en ce que** le vieillissement se fait à une température supérieure à 60 °C, particulièrement à une température s'inscrivant dans une plage allant de 65 à 80 °C.

13. Procédé selon l'une quelconque des revendications précédentes, **caractérisé en ce que** l'étape de précipitation est réalisée par précipitation en continu.

14. Procédé selon l'une quelconque des revendications précédentes, **caractérisé en ce que** la première solution de cuivre et zinc est obtenue dans une proportion choisie entre 1:99 et 99:1.

15. Procédé selon l'une quelconque des revendications précédentes, **caractérisé en ce que** le précipité est calciné après le séchage.

16. Procédé selon la revendication 15, dans lequel le précipité est calciné à des températures s'inscrivant dans une plage allant de 140 à 1 000 °C, de préférence de 170 à 350 °C pour une durée d'au moins 0,1 seconde, de préférence 20 minutes à 8 heures, de manière particulièrement préférée de 30 minutes à 4 heures.

17. Procédé selon l'une quelconque des revendications précédentes, **caractérisé en ce que** l'eau résiduelle contient des ions formiates et que l'eau résiduelle contenant du formiate est soumise à un traitement par oxydation, les ions formiates s'oxydant sensiblement en carbonate, hydrogénocarbonate, dioxyde de carbone et eau.

18. Procédé selon la revendication 17, **caractérisé en ce que** le peroxyde d'hydrogène est ajouté à l'eau résiduelle contenant du formiate en vue du traitement par oxydation.

19. Procédé selon la revendication 17 ou 18, **caractérisé en ce que** le traitement par oxydation de l'eau résiduelle contenant du formiate se fait avant la séparation du précipité.

20. Procédé selon l'une quelconque des revendications 17 à 19, **caractérisé en ce que** le traitement par oxydation se fait par un traitement biologique de l'eau résiduelle contenant du formiate.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1965007
- DE 2302658 A
- DE 2056612 A
- US 4279781 A
- EP 0125689 A2
- EP 0152809 A2
- WO 03053569 A1
- JP 2001144779 A